# EUROPEAN PATENT SPECIFICATION

(11) **EP 2 389 550 B1**
(45) Date of publication and mention of the grant of the patent: **28.06.2017**
(21) Application number: 09838924.0
(22) Date of filing: 20.11.2009
(51) Int. Cl.: F25D 17/06

(54) **REFRIGERATOR RELATED TECHNOLOGY**
KÜHLVORRICHTUNGEN BETREFFENDE TECHNOLOGIE
TECHNOLOGIE RELATIVE AUX RÉFRIGÉRATEURS

(30) Priority: 21.01.2009 KR 20090005007
(43) Date of publication of application: 30.11.2011
(73) Proprietor: LG Electronics Inc., Seoul 150-721 (KR)
(72) Inventor: LEE, Youn Seok, Seoul 153-802 (KR); LEE, Jang Seok, Seoul 153-802 (KR); OH, Min Kyu, Seoul 153-802 (KR); KIM, Kyeong Yun, Seoul 153-802 (KR); CHAE, Su Nam, Seoul 153-802 (KR)
(74) Representative: Cabinet Plasseraud
(86) International application number: PCT/KR2009/006862
(87) International publication number: WO 2010/085037

(56) References cited:
- JP-A- 3 102 169
- JP-A- H03 102 169
- US-A- 3 712 078
- US-A- 3 712 078
- US-A- 4 879 881

## Description

### Technical Field

The present disclosure related to refrigerator technology.

### Background Art

A refrigerator is used to supply cold air generated at an evaporator to a storage compartment (e.g., a refrigerating and/or freezing compartment) to maintain freshness of various food products stored in the storage compartment. Such a refrigerator includes a body, in which a storage compartment is defined to store food at a low-temperature state. A door is mounted to a front side of the body to open or close the storage compartment.

A cooling cycle is included in the refrigerator to cool the storage compartment through circulation of a refrigerant. A machine compartment is also defined in the body to accommodate a plurality of electric elements used to configure the cooling cycle.

For instance, the cooling cycle includes a compressor to perform a temperature/pressure increasing operation upon a low-temperature/low-pressure gaseous refrigerant such that the low-temperature/low-pressure gaseous refrigerant is changed into a high-temperature/highpressure gaseous refrigerant. The cooling cycle also includes a condenser to condense the refrigerant supplied from the compressor, using ambient air, an expansion valve to perform a pressure reducing operation upon the refrigerant supplied from the condenser such that the refrigerant is expanded, and an evaporator to evaporate the refrigerant emerging from the expansion valve in a low pressure state, thereby absorbing heat from the interior of the refrigerator. Examples of related art can be found in JPH03102169A, US4879881A and US3712078A. Japanese patent application discloses a high-humidity and low-temperature storage cabinet that should protect the stored food from dew droplets and sanitarily control it. A refrigerator according to the preamble of independent claim 1 is known from US4879881. US3712078A relates to a refrigerating unit for a refrigerator with a chamber and covers that is capable of being bodily mounted in refrigerators of different designs.

### Disclosure of Invention

### Technical Problem

There is a reduction in heat exchange efficiency due to flow resistance.

### Solution to Problem

Accordingly, the present invention is directed to a refrigerator that substantially obviates one or more problems due to limitations and disadvantages of the related art.
An object of the present invention is to provide a refrigerator in which constituent elements of a cooling cycle do not extend into a storage compartment.
Another object of the present invention is to provide a refrigerator configured to reduce flow resistance of cold air at a constant heat exchange area of an evaporator.
Additional advantages, objects, and features of the invention will be set forth in part in the description which follows and in part will become apparent to those having ordinary skill in the art upon examination of the following or may be learned from practice of the invention. The objectives and other advantages of the invention may be realized and attained by the structure particularly pointed out in the written description and claims hereof as well as the appended drawings.

A refrigerator includes a body, a storage compartment defined in a first portion of the body, a door configured to open and close at least a portion of the storage compartment, and a cold air generating compartment defined in an upper portion of the body and configured to supply cold air to the storage compartment. The upper portion of the body is positioned above the storage compartment when the refrigerator is oriented in an ordinary operating orientation. The refrigerator also includes an evaporator positioned in the cold air generating compartment and a cold air fan positioned in the cold air generating compartment and configured to promote movement of air within the cold air generating compartment in a flow direction that passes over the evaporator and is perpendicular to a surface of the door when the door is oriented in a closed position. The refrigerator further includes a guide duct arranged in the body to connect the storage compartment and the cold air generating compartment and configured to guide air flow between the storage compartment and the cold air generating compartment.

The cold air generating compartment extends across a depth of the body from a front side of the body to a rear side of the body and the guide duct is configured to guide air flowing from the rear side of the storage compartment to a rear side of the cold air generating compartment and air flowing from a front side of the cold air generating compartment to the rear side of the storage compartment.

The guide duct defines a first flow path that guides the air flowing from the rear side of the storage compartment to the rear side of the cold air generating compartment and a second flow path that guides the air flowing from the front side of the cold air generating compartment to the rear side of the storage compartment. The first and second flow paths are preferably separated from each other.

Further, the refrigerator includes a first duct configured to guide the air flowing from the rear side of the storage compartment to the rear side of the cold air generating compartment and a second duct configured to guide the air flowing from the front side of the cold air generating compartment to the rear side of the storage compartment. The first duct is arranged at the rear side of the storage compartment and the second duct is arranged to extend along a top wall of the storage compartment and the rear side of the storage compartment.

The second duct includes an inlet portion arranged at a front side of the top wall of the storage compartment and configured to receive air from the cold air generating compartment and a guide portion arranged at one side of the top wall of the storage compartment, connected to the inlet portion, and configured to guide, within the top wall, cold air received by the inlet portion to the rear side of the storage compartment without entering the storage compartment at the top wall. The second duct also includes a discharge portion arranged at the rear side of the storage compartment, connected to the guide portion, and configured to guide cold air from the guide portion into the storage compartment at the rear side of the storage compartment. The portions of the second duct arranged at the top wall of the storage compartment may be arranged at a bottom wall of the cold air generating compartment. The portion of the second duct arranged at the rear side of the storage compartment is parallel to and separated from the first duct.

In addition, the refrigerator may include a cold air inlet defined at the first duct and configured to receive cold air from the storage compartment and a cold air outlet defined at the second duct and configured to discharge cold air into the storage compartment. The evaporator may have a vertical length perpendicular to the flow direction of cold air along the evaporator and a horizontal length parallel to the flow direction of cold air such that the vertical length is longer than the horizontal length.

The cold air fan may be arranged in the front or rear of the evaporator in the cold air generating compartment and is configured to guide cold air received from the first duct such that the cold air flows to the second duct after passing through the evaporator. The cold air fan may include one of a centrifugal fan, an axial fan, and a cross-flow fan. The cold air fan may be configured to propel the cold air toward an inlet of the second duct.

The first duct preferably communicates with a rear side of a bottom wall of the cold air generating compartment and the second duct may communicate with a front side of the bottom wall of the cold air generating compartment. The refrigerator preferably include an inlet defined at the first duct and configured to guide air from the storage compartment to the first duct and an outlet defined at the second duct and configured to guide air from the second duct to the storage compartment.

In addition, the second duct includes an inlet portion arranged at a front side of the top wall of the storage compartment and configured to receive air from the cold air generating compartment. The second guide duct also includes a guide portion arranged at one side of the top wall of the storage compartment, connected to the inlet portion, and configured to guide cold air from the inlet portion to the rear side of the storage compartment without entering the storage compartment at the top wall. The second guide duct further includes a discharge portion arranged at the rear side of the storage compartment, connected to the guide portion, and configured to guide cold air from the guide portion into the storage compartment at the rear side of the storage compartment.

The refrigerator includes a door configured to open and close at least a portion of the storage compartment. The cold air fan is configured to promote movement of air within the cold air generating compartment in a flow direction that passes over the evaporator and is perpendicular to a surface of the door when the door is oriented in a closed position. The evaporator preferably has a vertical length perpendicular to the flow direction of cold air along the evaporator and a horizontal length parallel to the flow direction of cold air such that the vertical length is longer than the horizontal length.

### Advantageous Effects of Invention

In accordance with the configurations of the first and second ducts the flow path of cold air introduced from the storage compartment into the cold air generating compartment and the flow path of cold air introduced into the storage compartment after being discharged out of the cold air generating compartment are preferably separate from each other.

Further, heat exchange is performed in the cold air generating compartment arranged at the upper portion of the body. Because the cold air generating compartment extends in forward and rearward directions of the body and the evaporator and cold air fan are installed in an aligned state in a longitudinal direction of the cold air generating compartment , the guide duct does not extend into the storage compartment even though it is arranged between the storage compartment and the cold air generating compartment.

The evaporator , cold air fan , and guide duct are installed without regard for the height of the cold air generating compartment because the evaporator and cold air fan are not arranged in a vertical direction, but arranged in forward and rearward directions.

Also, the evaporator is configured such that the length thereof perpendicular to the flow direction of cold air along the evaporator is longer than the length w thereof parallel to the flow direction of cold air.

In the evaporator having the above-described structure, the length of a flow path, through which cold air flows along the evaporator , is reduced for a constant heat exchange area, as compared to a structure in which the length of the evaporator perpendicular to the flow direction of cold air is shorter than the horizontal length of the evaporator parallel to the flow direction of cold air. As a result, the flow resistance of cold air is reduced, as compared to the latter structure.

Even if the guide duct, which defines the cold air introduction and discharge paths, is not arranged at the front side of the body , but arranged at the rear side of the body , the door opening and closing operations of the refrigerator does not interfere with the circulation of cold air.

### Brief Description of Drawings

FIG. 1 is a partial sectional view illustrating a part of a configuration of a refrigerator according to the prior art;
FIG. 2 is a perspective view illustrating a configuration of a refrigerator according to the invention;
FIGs. 3 and 4 are a side view and a sectional view illustrating a configuration of a refrigerator according to the invention;
FIG. 5 is a sectional view illustrating a configuration of a refrigerator according to the invention; and
FIG. 6 is a sectioned perspective view schematically illustrating the structure of a guide duct.

### Best Mode for Carrying out the Invention

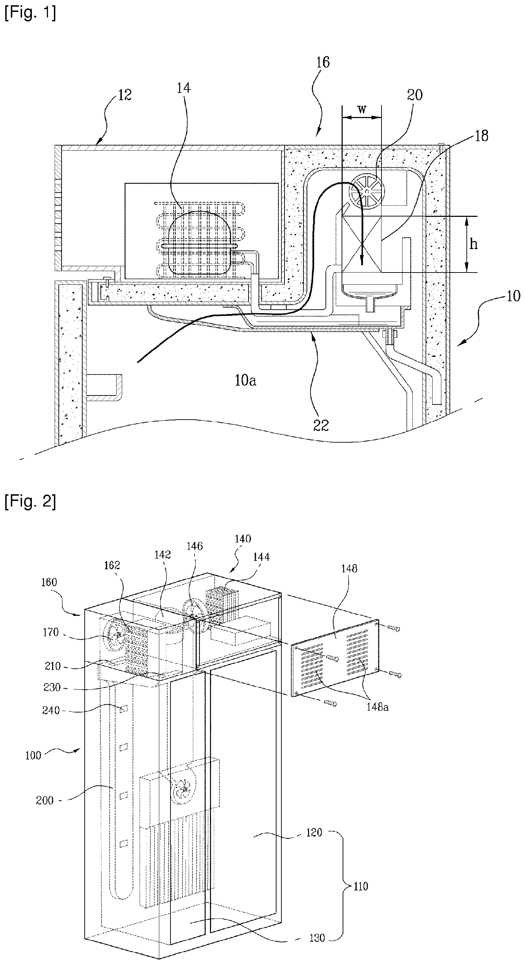

FIG. 1 illustrates an example refrigerator. As shown in FIG. 1, the refrigerator includes a body 10. A storage compartment 10a is defined in the body 10 to store food therein. A machine compartment 12 is defined in an upper portion of the body 10. The machine compartment 12 accommodates a plurality of electric elements used in a cooling cycle including a compressor 14 to compress a refrigerant, etc.

A cold air generating compartment 16 also is defined in the upper portion of the body 10 at one side of the machine compartment 12. An evaporator 18 that generates cold air through heat exchange is arranged in the cold air generating compartment 16. According to a preferred embodiment, the evaporator 18 is configured such that a vertical length thereof (h) is longer than a lateral length thereof (w).

A cold air fan 20 is arranged over the evaporator 18. The cold air fan 20 draws cold air from the storage compartment 10a in order to allow the cold air to heat-exchange with the evaporator 18. A guide duct 22 defines a flow path of cold air and is arranged beneath the evaporator 18.

In the refrigerator shown in FIG. 1, the cold air generating compartment 16 extends in a vertical direction for a relatively long length because the evaporator 18 and cold air fan 20 are vertically arranged. As a result, the guide duct 22, etc. arranged beneath the cold air generating compartment 16 protrude into the storage compartment 10a. Where the guide duct 22, etc. protrude into the storage compartment 10a, as mentioned above, the capacity of a storage compartment of the refrigerator is reduced.

In addition, the evaporator 18 has an enhanced heat exchange efficiency when it has an increased cold air introduction area at a front side thereof. In the refrigerator shown in FIG. 1, increasing the cold air introduction area at the front side of the evaporator 18 facing the cold air fan 20 has limitations because a space allowing cold air to flow along the evaporator 18 is provided and the cold air fan 20 is arranged over the evaporator 18. That is, the evaporator 18 has a length (w) perpendicular to a flow direction of cold air along the evaporator 18 and a length (h) parallel to the flow direction of cold air such that the length (w) is shorter than the length (h). As a result, a reduction in heat exchange efficiency due to flow resistance may exist.

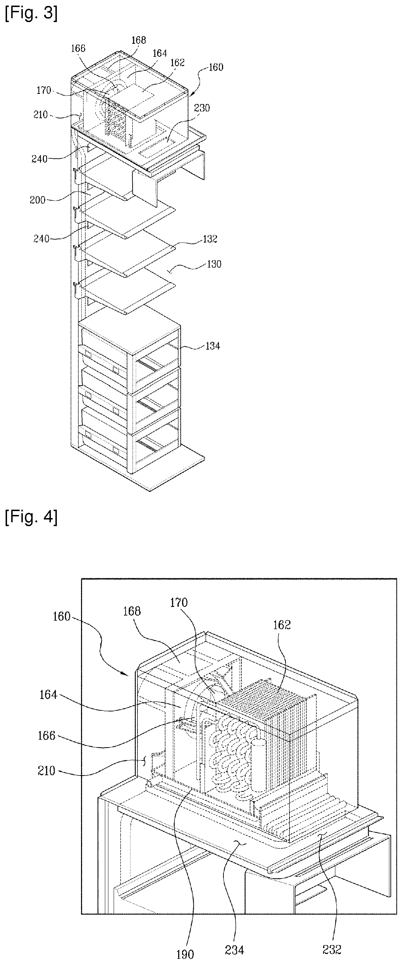

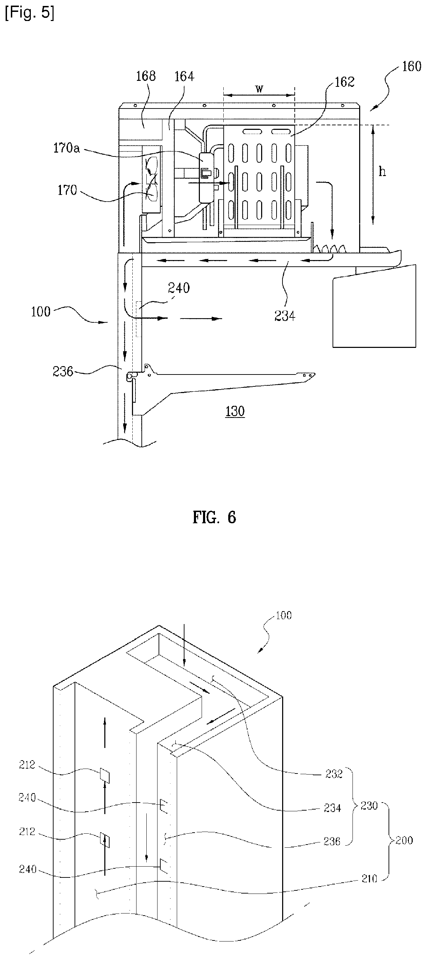

FIG. 2 illustrates a refrigerator according to the invention. FIGs. 3, 4 and 5 illustrate the refrigerator shown in FIG. 2. FIG. 6 illustrates the structure of a guide duct.

As shown in the drawings, in a body 100 that defines an appearance and a frame of the refrigerator, a storage compartment 110 is defined. The storage compartment 110 is a space to store food therein. The storage compartment 110 is divided into a refrigerating compartment 120 and a freezing compartment 130. A plurality of racks 132 are vertically arranged in the storage compartment 110. A drawer type storage compartment 134 also is defined beneath the racks.

A machine compartment 140 is defined in an upper portion of the body 100. The machine compartment 140 accommodates one or more elements of a refrigeration cycle. Accommodated in the machine compartment 140 are a compressor 142 to perform a temperature/pressure increasing operation upon a low-temperature/low-pressure gaseous refrigerant such that the low-temperature/low-pressure gaseous refrigerant is changed into a high-temperature/high-pressure gaseous refrigerant. A condenser 144 to condense the refrigerant supplied from the compressor 142, using ambient air, an expansion valve to perform a pressure reducing operation upon the refrigerant supplied from the condenser 144 such that the refrigerant is expanded, and a blowing fan 146 to draw in ambient air, thereby cooling the condenser 144, are also accommodated in the machine compartment 140. The machine compartment 140 is screened by a cover member 148 that has at least one through hole 148a.

As shown in FIGs. 3 and 5, a cold air generating compartment 160 is defined in an upper portion of the body 100 at one side of the machine compartment 140. The cold air generating compartment 160 is a space in which a configuration to generate cold air to maintain the storage space at low temperature is installed. The cold air generating compartment 160 is separated from the storage compartment 110 by one or more walls.

The cold air generating compartment 160 extends from a front side of the body 100 to a rear side of the body 100. In the cold air generating compartment 160, an evaporator 162 and a cold air fan 170 are horizontally arranged. The evaporator 162 absorbs heat from the surroundings when a refrigerant emerging from the expansion valve is evaporated in a low temperature state.

The cold air generating compartment extends in a horizontal direction. Cold air is introduced into the cold air generating compartment 160 at a front side of the cold air generating compartment 160, and is discharged out of the cold air generating compartment 160 at a rear side of the cold air generating compartment 160. Accordingly, the evaporator 162 can extend lengthily in a vertical direction of the cold air generating compartment 160. That is, the evaporator 162 has a length h perpendicular to a flow direction of cold air along the evaporator 162 and a length w parallel to the flow direction of cold air such that the length h is longer than the length w.

An orifice 164, which has an orifice hole 166, is arranged around the evaporator 162. A guide member 168 is arranged at one side of a top of the orifice hole 166. The guide member 168 guides cold air emerging from the storage compartment 110 to the cold air fan 170.

The cold air fan 170 is arranged at the rear of the evaporator 162 in the cold air generating compartment 160 to guide cold air emerging from a first duct 210 such that the cold air flows across the evaporator 162 to a second duct 230. The cold air fan 170 may be one of a centrifugal fan, an axial fan, or a cross-flow fan, to move (e.g., expel) cold air toward an inlet of the second duct 230. As shown, the cold air fan 170 is arranged in the rear of the cold air generating compartment 160 and evaporator 162. In other examples, the cold air fan 170 may be arranged in the front of the cold air generating compartment 160 and evaporator 162 in accordance with design conditions.

A cold air fan motor 170a that drives the cold air fan 170 is provided at the orifice 164 (FIG. 5). The cold air fan motor 170a is arranged at an extension from one side of the orifice 164.

Cold air is introduced into the cold air generating compartment 160 at the rear side of the cold air generating compartment 160, and is discharged out of the cold air generating compartment 160 at the front side of the cold air generating compartment 160. The rear side of the cold air generating compartment 160 communicates with the first duct 210. The front side of the cold air generating compartment 160 communicates with the second duct 230. A drain pan 190 is arranged beneath the evaporator 162 to collect defrost water generated during a defrosting operation and then to outwardly discharge the collected defrost water.

In addition, as shown in FIG. 6, a guide duct 200 is provided at the body 100. The guide duct 200 is arranged adjacent to the storage compartment 110. The guide duct 200 communicates with the storage compartment 110 and cold air generating compartment 160 to define a cold air circulation path.

The guide duct 200 includes first and second ducts 210 and 230. The first duct 210 extends in a vertical direction at a rear side of the body 100 to define a flow path that guides cold air to the cold air generating compartment 160.

Cold air inlets 212 are provided at the first duct 210. The cold air inlets 212 guide cold air from the storage compartment 110 to be introduced into the first duct 210.

The second duct 230 is parallel, at a certain portion thereof, to the first duct 210. The second duct 230 defines a flow path that guides cold air emerging from the cold air generating compartment 160 to the storage compartment 110.

The second duct 230 includes an inlet portion 232, into which cold air emerging from the cold air fan 170 is introduced, a guide portion 234 that defines a flow path for guiding the cold air introduced into the inlet portion 232 to flow in a forward/ rearward direction of the body 100, and a discharge portion 236 connected to the guide portion 234 extends in parallel to the first duct 210 and discharges the cold air to the storage compartment 110.

The inlet portion 232 corresponds to the inlet of the second duct 230. The inlet portion 232 extends along an edge of the front side of the body 100. The guide portion 234 extends in a forward/rearward direction at one side of the top of the body 100 while being flush with the inlet portion 232.

Since the evaporator 162 and cold air fan 170 are arranged in the cold air generating compartment 160, the guide portion 234 is arranged at one side of the bottom of the cold air generating compartment 160 in order to prevent the guide portion 234 from interfering with the evaporator 162 and cold air fan 170. The discharge portion 236 is separate from the first duct 210. The discharge portion 236 extends in the vertical direction of the body 100, similar to the first duct 210.

Cold air outlets 240 are provided at the second duct 230. The cold air outlets 240 guide cold air cooled to a low temperature while passing along the evaporator 162 such that the cold air is again introduced into the storage compartment 110.

Operation of the refrigerator having the above-described configuration is described with reference to FIGS. 5 and 6.

In the body 100, cold air present in the storage compartment 110 is introduced into the cold air generating compartment 160 after flowing through the cold air inlets 212 and first duct 210. The cold air is cooled in the cold air generating compartment 160 in accordance with heat exchange thereof with the evaporator 162. The cold air is then again introduced into the storage compartment 110 after passing through the second duct 230. For instance, the cold air passes through the inlet portion 232, the guide portion 234, the discharge portion 236, and out of the second duct 230 through the cold air outlets 240.

In accordance with the configurations of the first and second ducts 210 and 220, the flow path of cold air introduced from the storage compartment 110 into the cold air generating compartment 160 and the flow path of cold air introduced into the storage compartment 110 after being discharged out of the cold air generating compartment 160 may be separate from each other.

Further, heat exchange is performed in the cold air generating compartment 160 arranged at the upper portion of the body 100. Because the cold air generating compartment 160 extends in forward and rearward directions of the body 100 and the evaporator 162 and cold air fan 170 are installed in an aligned state in a longitudinal direction of the cold air generating compartment 160, the guide duct 200 does not extend into the storage compartment 110 even though it is arranged between the storage compartment 110 and the cold air generating compartment 160. The evaporator 162, cold air fan 170, and guide duct 200 are installed without regard for the height of the cold air generating compartment 160 because the evaporator 162 and cold air fan 170 are not arranged in a vertical direction, but arranged in forward and rearward directions.

Also, the evaporator 162 is configured such that the length h thereof perpendicular to the flow direction of cold air along the evaporator 162 is longer than the length w thereof parallel to the flow direction of cold air. In the evaporator 162 having the above-described structure, the length of a flow path, through which cold air flows along the evaporator 162, is reduced for a constant heat exchange area, as compared to a structure in which the length of the evaporator perpendicular to the flow direction of cold air is shorter than the horizontal length of the evaporator parallel to the flow direction of cold air. As a result, the flow resistance of cold air is reduced, as compared to the latter structure.

Even if the guide duct 200, which defines the cold air introduction and discharge paths, is not arranged at the front side of the body 100, but arranged at the rear side of the body 100, the door opening and closing operations of the refrigerator does not interfere with the circulation of cold air.

## Claims

1. A refrigerator comprising:
a body (100);
a storage compartment (110) defined in a first portion of the body (100);
a door configured to open and close at least a portion of the storage compartment (110);
a cold air generating compartment (160) defined in an upper portion of the body (100) and configured to supply cold air to the storage compartment (110), the upper portion of the body (100) being positioned above the storage compartment (110) when the refrigerator is oriented in an ordinary operating orientation, the cold air generating compartment (160) extending across a depth of the body (100) from a front side of the body (100) to a rear side of the body (100);
an evaporator (162) positioned in the cold air generating compartment (160);
a cold air fan (170) positioned in the cold air generating compartment (160) and configured to promote movement of air within the cold air generating compartment (160) in a flow direction that passes over the evaporator (162) and is perpendicular to a surface of the door when the door is oriented in a closed position; and
a guide duct (200) arranged in the body (100) to connect the storage compartment (110) and the cold air generating compartment (160) and configured to guide air flow between the storage compartment (110) and the cold air generating compartment (160),wherein
the guide duct (200) comprises:
a first duct (210) arranged at the rear side of the storage compartment (110) and configured to guide the air flowing from the rear side of the storage compartment (110) to the rear side of the cold air generating compartment (160); and
a second duct (230) arranged to extend along a top wall of the storage compartment (110) and the rear side of the storage compartment (110) and configured to guide the air flowing from the front side of the cold air generating compartment (160) to the rear side of the storage compartment (110), wherein
the second duct (230) comprises:
an inlet portion (232) arranged at the top wall of the storage compartment (110);
a guide portion (234) arranged at one side of the top wall of the storage compartment (110), and configured to guide, within the top wall, cold air received by the inlet portion (232) to the rear side of the storage compartment (110) without entering the storage compartment (110) at the top wall; and
a discharge portion (236) arranged at the rear side of the storage compartment (110), connected to the guide portion (234), and configured to guide cold air from the guide portion (234) into the storage compartment (110) at the rear side of the storage compartment (110),
the discharge portion (236) of the second duct (230) is parallel to the first duct (210) and is separated from the first duct (210),
the inlet portion (232) is configured to receive air from the cold air generating compartment (160), and
the guide portion (234) is connected to the inlet portion (232), **characterized in that** the inlet portion (232) is arranged at a front side of the top wall of the storage compartment (110).

2. The refrigerator according to claim 1, wherein the first duct (210) and the second duct (230) are separated from each other.

3. The refrigerator according to claim 1 or 2, wherein the portions of the second duct (230) arranged at the top wall of the storage compartment (110) are arranged at a bottom wall of the cold air generating compartment (160).

4. The refrigerator according to any one of claims 1 to 3, further comprising:
a cold air inlet (212) defined at the first duct (210) and configured to receive cold air from the storage compartment (110) ; and
a cold air outlet (240) defined at the second duct (230) and configured to discharge cold air into the storage compartment (110).

5. The refrigerator according to any one of claims 1 to 4, wherein the evaporator (162) has a vertical length perpendicular to the flow direction of cold air along the evaporator (162) and a horizontal length parallel to the flow direction of cold air such that the vertical length is longer than the horizontal length.

6. The refrigerator according to any one of claims 1 to 5, wherein the cold air fan (146) is arranged in the front or rear of the evaporator (162) in the cold air generating compartment (160) and is configured to guide cold air received from the first duct (210) such that the cold air flows to the second duct (230) after passing through the evaporator (162).

7. The refrigerator according to any one of claims 1 to 6, wherein the cold air fan (146) comprises one of a centrifugal fan, an axial fan, and a cross-flow fan.

8. The refrigerator according to claim 7, wherein the cold air fan (146) is configured to propel the cold air toward the inlet portion (232) of the second duct (230).

## Patentansprüche

1. Kühlvorrichtung aufweisend:
einen Körper (100);
eine Aufbewahrungskammer (110), die in einem ersten Abschnitt des Körpers (100) definiert ist;
eine Tür zum Öffnen und Schließen mindestens eines Abschnitts der Aufbewahrungskammer (110);
eine Kaltlufterzeugungskammer (160), die in einem oberen Abschnitt des Körpers (100) definiert ist und konfiguriert ist, der Aufbewahrungskammer (110) Kaltluft zuzuführen, wobei der obere Abschnitt des Körpers (100) oberhalb der Aufbewahrungskammer (110) angeordnet ist, wenn die Kühlvorrichtung in einer üblichen Gebrauchsorientierung orientiert ist, wobei die Kaltlufterzeugungskammer (160) sich von einer Vorderseite des Körpers (100) über eine Tiefe des Körpers (100) zu einer Rückseite des Körpers (100) erstreckt;
einen Verdampfer (162), der in der Kaltlufterzeugungskammer (160) angeordnet ist;
einen Kaltluftventilator (170), der in der Kaltlufterzeugungskammer (160) angeordnet ist und konfiguriert ist, im Innern der Kaltlufterzeugungskammer (160) eine Luftbewegung in eine Strömungsrichtung zu befördern, die den Verdampfer (162) überquert und senkrecht zu einer Oberfläche der Tür ist, wenn die Tür in einer geschlossenen Position orientiert ist; und
einen Führungskanal (200), der in dem Körper (100) angeordnet ist, um die Aufbewahrungskammer (110) und die Kaltlufterzeugungskammer (160) zu verbinden, und der konfiguriert ist, einen Luftstrom zwischen der Aufbewahrungskammer (110) und der Kaltlufterzeugungskammer (160) zu führen, wobei
der Führungskanal (200) aufweist:
einen ersten Kanal (210), der an der Rückseite der Aufbewahrungskammer (110) angeordnet ist und konfiguriert ist, die von der Rückseite der Aufbewahrungskammer (110) zu der Rückseite der Kaltlufterzeugungskammer (160) strömende Luft zu führen; und
einen zweiten Kanal (230), der angeordnet ist, um sich entlang einer oberen Wand der Aufbewahrungskammer (110) und der Rückseite der Aufbewahrungskammer (110) zu erstrecken, und der konfiguriert ist, die von der Vorderseite der Kaltlufterzeugungskammer (160) zu der Rückseite der Aufbewahrungskammer (110) strömende Luft zu führen, wobei
der zweite Kanal (230) aufweist:
einen Einlassabschnitt (232), der an der oberen Wand der Aufbewahrungskammer (110) angeordnet ist;
einen Führungsabschnitt (234), der an einer Seite der oberen Wand der Aufbewahrungskammer (110) angeordnet ist und konfiguriert ist, von dem Einlassabschnitt (232) empfangene Kaltluft innerhalb der oberen Wand zu der Rückseite der Aufbewahrungskammer (110) zu führen, ohne dass sie an der oberen Wand in die Aufbewahrungskammer (110) eintritt; und
einen Auslassabschnitt (236), der an der Rückseite der Aufbewahrungskammer (110) angeordnet ist, mit dem Führungsabschnitt (234) verbunden ist und konfiguriert ist, an der Rückseite der Aufbewahrungskammer (110) Kaltluft aus dem Führungsabschnitt (234) in die Aufbewahrungskammer (110) zu führen,
wobei der Auslassabschnitt (236) des zweiten Kanals (230) parallel zu dem ersten Kanal (210) ist und von dem ersten Kanal (210) getrennt ist,
der Einlassabschnitt (232) konfiguriert ist, Luft aus der Kaltlufterzeugungskammer (160) zu empfangen, und
der Führungsabschnitt (234) mit dem Einlassabschnitt (232) verbunden ist,
**dadurch gekennzeichnet, dass** der Einlassabschnitt (232) an einer Vorderseite der oberen Wand der Aufbewahrungskammer (110) angeordnet ist.

2. Kühlvorrichtung nach Anspruch 1, wobei der erste Kanal (210) und der zweite Kanal (230) voneinander getrennt sind.

3. Kühlvorrichtung nach Anspruch 1 oder 2, wobei die an der oberen Wand der Aufbewahrungskammer (110) angeordneten Abschnitte des zweiten Kanals (230) an einer unteren Wand der Kaltlufterzeugungskammer (160) angeordnet sind.

4. Kühlvorrichtung nach einem der Ansprüche 1 bis 3, ferner aufweisend:
einen Kaltlufteinlass (212), der an dem ersten Kanal (210) definiert ist und konfiguriert ist, Kaltluft aus der Aufbewahrungskammer (110) zu empfangen; und
einen Kaltluftauslass (240), der an dem zweiten Kanal (230) definiert ist und konfiguriert ist, Kaltluft in die Aufbewahrungskammer (110) strömen zu lassen.

5. Kühlvorrichtung nach einem der Ansprüche 1 bis 4, wobei der Verdampfer (162) eine vertikale Länge senkrecht zu der Strömungsrichtung der Kaltluft entlang des Verdampfers (162) und eine horizontale Länge parallel zu der Strömungsrichtung der Kaltluft hat, derart, dass die vertikale Länge länger als die horizontale Länge ist.

6. Kühlvorrichtung nach einem der Ansprüche 1 bis 5, wobei der Kaltluftventilator (146) in der Kaltlufterzeugungskammer (160) vor oder hinter dem Verdampfer (162) angeordnet ist und konfiguriert ist, aus dem ersten Kanal (210) empfangene Kaltluft zu führen, so dass die Kaltluft nach Durchgang durch den Verdampfer (162) zu dem zweiten Kanal (230) strömt.

7. Kühlvorrichtung nach einem der Ansprüche 1 bis 6, wobei der Kaltluftventilator (146) einen Zentrifugalventilator oder einen Axialventilator oder einen Querstromventilator aufweist.

8. Kühlvorrichtung nach Anspruch 7, wobei der Kaltluftventilator (146) konfiguriert ist, Kaltluft zu dem Einlassabschnitt (232) des zweiten Kanals (230) hin zu treiben.

## Revendications

1. Réfrigérateur comprenant :
un corps (100) ;
un compartiment de stockage (110) défini dans une première portion du corps (100) ;
une porte configurée pour ouvrir et fermer au moins une portion du compartiment de stockage (110) ;
un compartiment de génération d'air froid (160) défini dans une portion supérieure du corps (100) et configuré pour fournir de l'air froid au compartiment de stockage (110), la portion supérieure du corps (100) étant positionnée au-dessus du compartiment de stockage (110) lorsque le réfrigérateur est orienté dans une orientation de fonctionnement ordinaire, le compartiment de génération d'air froid (160) s'étendant à travers une profondeur du corps (100) d'un côté avant du corps (100) à un côté arrière du corps (100) ;
un évaporateur (162) positionné dans le compartiment de génération d'air froid (160) ;
un ventilateur d'air froid (170) positionné dans le compartiment de génération d'air froid (160) et configuré pour favoriser un mouvement d'air au sein du compartiment de génération d'air froid (160) dans une direction d'écoulement qui passe par-dessus l'évaporateur (162) et est perpendiculaire à une surface de la porte lorsque la porte est orientée dans une position fermée ; et
une conduite de guidage (200) agencée dans le corps (100) pour raccorder le compartiment de stockage (110) et le compartiment de génération d'air froid (160) et configurée pour guider un écoulement d'air entre le compartiment de stockage (110) et le compartiment de génération d'air froid (160), dans lequel la conduite de guidage (200) comprend :
une première conduite (210) agencée au niveau du côté arrière du compartiment de stockage (110) et configurée pour guider l'air s'écoulant du côté arrière du compartiment de stockage (110) au côté arrière du compartiment de génération d'air froid (160) ; et
une seconde conduite (230) agencée pour s'étendre le long d'une paroi haute du compartiment de stockage (110) et le côté arrière du compartiment de stockage (110) et configurée pour guider l'air s'écoulant du côté avant du compartiment de génération d'air froid (160) au côté arrière du compartiment de stockage (110), dans lequel
la seconde conduite (230) comprend :
une portion d'admission (232) agencée au niveau de la paroi haute du compartiment de stockage (110) ;
une portion de guidage (234) agencée au niveau d'un côté de la paroi haute du compartiment de stockage (110), et configurée pour guider, au sein de la paroi haute, de l'air froid reçu par la portion d'admission (232) vers le côté arrière du compartiment de stockage (110) sans entrer dans le compartiment de stockage (110) au niveau de la paroi haute ; et
une portion d'évacuation (236) agencée au niveau du côté arrière du compartiment de stockage (110), raccordée à la portion de guidage (234), et configurée pour guider de l'air froid de la portion de guidage (234) dans le compartiment de stockage (110) au niveau du côté arrière du compartiment de stockage (110) ;
la portion d'évacuation (236) de la seconde conduite (230) est parallèle à la première conduite (210) et est séparée de la première conduite (210),
la portion d'admission (232) est configurée pour recevoir de l'air du compartiment de génération d'air froid (160), et
la portion de guidage (234) est raccordée à la portion d'admission (232), **caractérisé en ce que** la portion d'admission (232) est agencée au niveau d'un côté avant de la paroi haute du compartiment de stockage (110).

2. Réfrigérateur selon la revendication 1, dans lequel la première conduite (210) et la seconde conduite (230) sont séparées l'une de l'autre.

3. Réfrigérateur selon la revendication 1 ou 2, dans lequel les portions de la seconde conduite (230) agencée au niveau de la paroi haute du compartiment de stockage (110) sont agencées au niveau d'une paroi basse du compartiment de génération d'air froid (160).

4. Réfrigérateur selon l'une quelconque des revendications 1 à 3, comprenant en outre :
une admission d'air froid (212) définie au niveau de la première conduite (210) et configurée pour recevoir de l'air froid du compartiment de stockage (110) ; et
un refoulement d'air froid (240) défini au niveau de la seconde conduite (230) et configuré pour évacuer de l'air froid dans le compartiment de stockage (110).

5. Réfrigérateur selon l'une quelconque des revendications 1 à 4, dans lequel l'évaporateur (162) a une longueur verticale perpendiculaire à la direction d'écoulement d'air froid le long de l'évaporateur (162) et une longueur horizontale parallèle à la direction d'écoulement d'air froid de telle sorte que la longueur verticale est plus longue que la longueur horizontale.

6. Réfrigérateur selon l'une quelconque des revendications 1 à 5, dans lequel le ventilateur d'air froid (146) est agencé à l'avant ou à l'arrière de l'évaporateur (162) dans le compartiment de génération d'air froid (160) et est configuré pour guider de l'air froid reçu de la première conduite (210) pour que l'air froid s'écoule vers la seconde conduite (230) après avoir traversé l'évaporateur (162).

7. Réfrigérateur selon l'une quelconque des revendications 1 à 6, dans lequel le ventilateur d'air froid (146) comprend l'un parmi un ventilateur centrifuge, un ventilateur axial et un ventilateur tangentiel.

8. Réfrigérateur selon la revendication 7, dans lequel le ventilateur d'air froid (146) est configuré pour propulser l'air froid vers la portion d'admission (232) de la seconde conduite (230).
